# EUROPEAN PATENT APPLICATION

(11) **EP 2 942 068 A1**
(43) Date of publication of application: **11.11.2015**
(21) Application number: 15163787.3
(22) Date of filing: 30.03.2010
(51) Int. Cl.: A61L 27/02, A61L 27/54, A61L 27/44

(54) **REGULATION OF BONE GROWTH USING ZEOLITE IN COMBINATION WITH BONE GRAFT SUBSTITUTES**

(30) Priority: 01.04.2009 US 211569 P; 01.03.2010 US 309143 P
(62) Divisional of application: 10759287.5
(71) Applicant: Difusion Technologies Inc., Georgetown, Texas 78726 (US)
(72) Inventor: Johns, Derrick, Austin, TX 78717 (US); Geck, Matthew, Austin, TX 78703 (US); Whang, Peter, Milford, CT 06461 (US); Hafiz, Jami, Cedar Park, TX 78613-4885 (US)
(74) Representative: UEXKÜLL & STOLBERG

(57) **Abstract**

Medical implants such as bone graft substitutes that include one or more cations are delivered in a local environment to promote osteogenesis. Zeolite loaded with a metal cation in combination with an implant such as a bone graft substitute can be used as an implant in the body to regulate protein transcription and translation. Also disclosed are methods of promoting osteogenesis in a patient in need thereof, methods for modulating bone formation and mineralization by implanting in a patient a medical implant comprising ion-exchangeable cations, and methods of regulating BMP gene expression in bone cells in a patient by controlling the delivery of certain cations through ionexchange via a zeolite incorporated in a bone substitute implanted in a patient so that BMP gene expression can be upregulated or downregulated appropriately.

## Description

This application claims priority of U.S. Provisional Application Serial No. 61/211,569 filed April 1, 2009 and U.S. Provisional Application Serial No. 61/309,143 filed March 1, 2010, the disclosures of which are incorporated herein by reference.

### BACKGROUND

Obtaining successful bone formation between different structures (e.g. fracture fragments, implants in bone) represents the primary objective of many orthopaedic, maxillofacial, and spinal procedures. As a result, it has been suggested that various techniques, proteins, and implants that promote osteogenesis may play a significant role in achieving successful outcomes following these surgeries.

Bone grafts are used to provide structural support, fill voids, and enhance the biologic repair of skeletal defects. Autogenous bone is harvested from an individual and transplanted into another location in the body to stimulate bone formation. The advantages of autograft are that it is the patient's own tissue and this material is associated with highest probability of successful healing.

However, this intervention may give rise to a number of complications such as infection, bleeding, chronic donor site pain, fracture and injury to neurovascular structures. Another disadvantage of autogenous bone graft is that it is only available in limited quantities. Allograft is bone that has been harvested from a cadaver which avoids much of the morbidity associated with autogenous bone. However, this tissue lacks the osteogenic properties of autograft and there is an inherent risk of either infectious disease transmission or an immunological reaction. Although these grafts have been shown to promote bone formation, both these strategies exhibit various shortcomings.

For these reasons, other materials that either assist or even replace autograft have been developed. Demineralized bone matrices (DBMs) are generated by the acid extraction of cortical bone; it has been suggested that removal of the mineral phase (i.e. the calcium and phosphate) may liberate osteoinductive proteins present in the matrix including the BMPs. DBMs are typically utilized with autogenous bone as a bone graft extender. Another class of bone graft extenders is ceramics and other synthetic materials.

By definition, an ideal bone graft must possess both excellent osteoconductive and osteoinductive properties when introduced into the surgical site. Osteoconductive scaffolds support the influx of osteogenic cells and the formation of new blood vessels which creates an environment conducive for bone growth. Osteoinductive proteins are capable of facilitating new bone formation by stimulating the differentiation of stem cells into osteoblasts. In order to be considered osteoinductive, a substance must contain one or more biologically active signaling molecules such that the concentration of these factors may ultimately determine whether a successful fusion will occur.

The BMPs are bioactive proteins that naturally occur in the human body and are regulated by various transcription and translation mechanisms. The BMPs belong to a family of growth factors that contribute to developmental processes such as pattern formation and tissue specification; in addition to inducing bone and cartilage formation, these proteins also regulate cell proliferation, migration, differentiation, and apoptosis in a number of tissues and organs. The BMPS have also been shown to promote wound healing and repair processes in adult tissues as well. A number of BMPs have been identified in humans and other animals including BMP-2, BMP-3 (osteogenin), BMP-3b (GDF-10), BMP-4 (BMP-2b), BMP-5, BMP-6, BMP-7 (osteogenic protein-1 or OP-1), BMP-8 (OP-2), BMP-8B (OP-3), BMP-9 (GDF-2), BMP-10, BMP-11 (GDF-11, BMP-12 (GDF-7), BMP-13 (GDF-6, CDMP-2), BMP-15 (GDF-9), BMP-16, GDF-1, GDF-3, GDF-5 (CDMP-1), and GDF-8 (myostatin). More recently, certain recombinant human BMPs have been approved by the Food and Drug Administration for limited clinical applications. For instance, INFUSE^{®} is a commercially available product that delivers rhBMP-2 in an absorbable collagen sponge which may be placed into titanium spacers for the purpose of interbody fusion in the lumbar spine.

The growth factors that modulate the transcription and subsequent translation of the BMPs are highly regulated through complex pathways. While osteogenesis may be augmented by the direct application of BMPs, this strategy has proven to be expensive and is not without its complications such as bone erosion, inflammatory seromas, and other adverse consequences.

Cationic zinc (Zn) is an important component involved in the process of bone growth. This is at least partially mediated by zinc fingers which are Zn-activated proteins that play an essential role in DNA recognition as well as the promotion, regulation, and expression of the bone morphogenetic proteins (BMPs) and other factors that regulate bone growth, healing, and fusion. Zinc fingers are one of the most abundant DNA-binding motifs that are comprised of a DNA-binding protein which resembles a finger with a base portion containing abundant cysteine and histidine amino acids that are bound to a zinc ion. These proteins may contain more than one finger in a single chain; each structure consists of 2 antiparallel beta-strands in conjunction with an alpha-helix. A single zinc ion is tetrahedrally coordinated by conserved histidine and cysteine residues, stabilizing the motif. The zinc ion is critical for the stability of the zinc finger protein because in the absence of this mineral the hydrophobic core becomes too small and the biologically active domain will unfold.

There is good evidence that zinc works to promote bone formation, mediated by Zinc Finger proteins that have multiple effects both upstream and downstream of the BMPs. It has previously been documented that increasing zinc levels in an animal model leads to greater osteogenesis characterized by bony growth, healing, and remodeling. Thus, based on these findings it would clearly be desirable to have an implant and a delivery system capable of regulating the optimum concentrations of zinc and other ions that are present in a local environment (e.g. spinal fusion bed) in order to promote osteogenesis in this particular area of interest.

### SUMMARY

The problems of the prior art have been overcome by the various embodiments disclosed herein, which provide devices such as medical implants such as those comprising bone graft substitutes that include one or more cations which are delivered in a local environment to promote osteogenesis. In certain embodiments, the absolute and relative concentrations of cations such as zinc, copper and/or silver at the site can be accurately regulated by varying the relative concentrations of the metals in a zeolite matrix incorporated in or applied to the implant as well as by varying the level of the zeolite in the implant. This flexibility allows the ion concentrations to be optimized for inhibition of microbial infection and biofilm formation, enhancing bone regeneration, and promoting efficient wound/tissue healing, for example. Zeolite in combination with an implant such as a bone graft substitute can be used in the body of a host to regulate protein transcription and translation. This therapeutic strategy will significantly affect the fields of orthopedics, spine surgery, maxillofacial surgery, cranial and neurosurgery, and other specialties.

In its method aspects, embodiments disclosed herein relate to promoting osteogenesis in a patient in need thereof. In certain embodiments, disclosed are methods for modulating bone formation and mineralization, comprising implanting in a host a medical implant comprising a source of cations, such as ion-exchangeable cations. In certain embodiments, the medical implant is bioactive. In certain embodiments, the source of cations is ion-exchangeable cations contained in a zeolite. In certain embodiments, disclosed are methods of regulating BMP gene expression in bone cells in a patient by controlling the delivery of certain cations through ion-exchange via a zeolite incorporated in an implant such as a bone substitute introduced in the patient. In accordance with certain embodiments, selective BMP gene expression can be upregulated (increased gene expression) or downregulated (inhibition or prevention of gene expression).

In certain embodiments, the implants are dental implants. A common problem with some dental implants is the lack of gum cell attachment resulting in "ditching". This causes the formation of blank pockets of space around the implant where bacteria proliferates leading to implant failure. Implant manufacturers typically focus on bone cell attachment but rarely gum cells (periodontal ligaments fibroblast and gingival fibroblast). BMPs can result in the proliferation of all the cells while at the same time the metal cations can reduce or eliminate harmful microbes.

### DETAILED DESCRIPTION

Embodiments disclosed herein relate to the use of zeolite as a cation cage in combination with intracorporeal devices, particularly medical implants such as bone growth substitutes to correctly deliver and dose one or more cations such as zinc, silver and/or copper as a tool to manage the pharmacokinetics of these cations in a local environment in order promote osteogenesis. The result is unique control of dosing of these cations in order to achieve a beneficial effect that often requires proper sustained dose for days or weeks while avoiding local cytotoxicity through controlled release.

The biologically active Zn Finger protein is an inherently unstable structure because its DNA helix is dependent upon a central zinc ion for its stability. By infusing zinc ions locally into the area in which bone formation is desired, the Zinc Finger can be stabilized which may affect physiologic gene transcription and up-regulation of the BMPs (e.g. BMP-2) and other osteogenic growth factors.

Suitable carriers for the source of cations include biocompatible matrices such as demineralized bone matrices, synthetic polymer matrices, or protein matrices such as collagen. It would be preferable for the carrier to be some type of bone growth substitute or osteopromotive agents that stimulate the formation of new bone via its osteoinductive and/or osteoconductive properties. Exemplary osteoconductive agents include collagen-based scaffolds such as Healos (a polymer-ceramic composite consisting of collagen fibers coated with hydroxyapatite and indicated for spinal fusions); glass-based scaffolds; silicate-based scaffolds; ceramic-based substitutes; polymer-based substitutes, allografts; calcium phosphates such as hydroxyapatite, tricalcium phosphate, or fluorapatite; calcium sulfate; demineralized bone matrix; or any combination thereof. Exemplary osteoinductive agents include bone morphogenetic proteins, demineralized bone matrix, various growth factors known to be osteoinductive (e.g., transforming growth factor-beta, growth and differentiation growth factor), stem cells or those with osteoblastic potential, etc. Other suitable bone graft substitutes are known to those skilled in the art, as exemplified by Helm, et al., "Bone Graft Substitutes for the Promotion of Spinal Arthrodesis", Neurosurg Focus, 10(4) (2001), the disclosure of which is hereby incorporated by reference.

In certain embodiments, the bone grafts are configured for use in spinal fusion (arthrodesis), such as for stabilizing an unstable spinal column due to structural deformity, trauma, degeneration, etc. Fusion is a surgical technique in which one or more vertebrae of the spine are united together ("fused") to reduce or eliminate relative motion between them or to fix the spatial relationship between them. Spinal fusions include posterolateral fusion, posterior lumbar interbody fusion, anterior lumbar interbody fusion, anterior/posterior spinal fusion, cervical fusion, thoracic fusion and interlaminar fusion. In certain embodiments, the bone grafts are for insertion in an intervertebral space between adjacent vertebrae. In certain embodiments, a fusion site is identified between adjacent vertebrae, a bone graft is implanted at said site, and a source of metal cations is presented at the fusion site in an amount effective for promoting osteogenesis. In certain embodiments, the implant is a spinal interbody cage, including cages comprising titanium, carbon fibers, biocompatible materials such as polyetheretherketone (PEEK), polyetherketoneketone (PEKK), or other synthetic substances. In certain embodiments, the metal ions are incorporated into the resin used to make the implant. For example, zeolite particles loaded with zinc, silver and/or copper ions are incorporated into the PEEK interbody cage. In certain embodiments, the cage is loaded with osteoconductive and/or osteoinductive agents, such as those disclosed above, to promote fusion.

In certain embodiments, intracorporeal devices include dental implants, appliances (e.g., orthodontic appliances) pegs, posts, caps, crowns, bridges and bridge reinforcements that include one or more cations which are delivered in a local environment to promote osteogenesis and/or wound tissue healing, and/or enhance bone regeneration, and/or inhibit microbial infection and/or biofilm formation. In certain embodiments, the absolute and relative concentrations of cations such as zinc, copper and/or silver at the site where the implant, appliance, peg, post, cap, crown, bridge or bridge reinforcement is placed can be accurately regulated by varying the relative concentrations of the metals in a zeolite matrix incorporated in or applied to the implant, appliance, peg, post, cap, crown, bridge or bridge reinforcement, as well as by varying the level of the zeolite therein. Zeolite in combination with such device can be used in the body of a host to regulate protein transcription and translation.

Such dental devices can be made of titanium, or more preferably a medical-grade polymer such as a polyetheretherketone (e.g., PEEK, commercially available as PEEK-OPTIMA from Invibio), which offers a combination of extensive biocompatibility, high strength, stiffness, toughness and good aesthetics. PEEK polymer exhibits almost the same combination of strength and biocompatibility, but without the negative effects attributed to metallics. The modulus of PEEK can be tailored to the bone, supporting natural tissue or natural teeth in the mouth, and thus can be used to develop metal-free dentures that do not restrict biting or chewing sensations. Additionally, the polymer easily can be coated and surface modified to enhance bone growth and osseointegration in accordance with the embodiments disclosed herein. The polymer will eliminate the electrical conductivity associated with titanium implants and since the polymer has excellent corrosion and acid resistance, it is ideal for long term usage in the demanding environment of the mouth.

Either natural zeolites or synthetic zeolites can be used to make the zeolites used in the embodiments disclosed herein. "Zeolite" is an aluminosilicate having a three dimensional skeletal structure that is represented by the formula: XM_{2/n}O•Al₂O₃•YsiO₂•ZH₂O, wherein M represents an ion-exchangeable ion, generally a monovalent or divalent metal ion, n represents the atomic valency of the (metal) ion, X and Y represent coefficients of metal oxide and silica respectively, and Z represents the number of water of crystallization. Examples of such zeolites include A-type zeolites, X-type zeolites, Y-type zeolites, T-type zeolites, high-silica zeolites, sodalite, mordenite, analcite, clinoptilolite, chabazite and erionite. The zeolite can be prepared by replacing some or all of the ion-exchangeable ions in zeolite (e.g., sodium ions, calcium ions, potassium ions, iron ions) with ammonium ions and zinc, silver and/or copper ions, such as is disclosed in U.S. Patent Nos. 4,939,958 and 4,911,898, the disclosure of which are hereby incorporated by reference. The amount of zinc, silver and/or copper ions in the zeolite should be sufficient such that they are present in an amount effective to promote osteogenesis over the time period required for bone replacement when combined with one or more osteogenerative agents, and implanted in the body. Exemplary amounts include from about 50 ppb to about 1000 ppb of zinc ions, and /or 20 ppb to about 200 ppb of copper and/or silver ions. Preferably the zinc is more prevalent than copper, generally about four times the concentration of copper. In certain embodiments, the metal cation is present at a level below the ion-exchange capacity in at least a portion of the zeolite particles.

In order to form the zeolite with the appropriate amount of ions, in certain embodiments the ions are incorporated into the solution by slurrying, for example, type A zeolite powder in water at 50 weight percent. Silver nitrate, copper nitrate and zinc nitrate with nitric acid are added to water.
Exemplary metal salt concentrations are between 1 and 10%. The metal ion solutions are poured into a mixing vessel, and the zeolite slurry is rapidly added to the vessel with strong agitation and the temperature taken to about 75°C. The slurry undergoes ion exchange for 1 to 24 hours. It is filtered and washed with distilled water. The slurry is dried at an appropriate temperature up to 200°C. When dry, the slurry is ground to an appropriate particle size. The relative concentrations of metals in solution will determine the loading ratio. The concentration, temperature and time of exchange will determine the overall loading of metals. Methods disclosed in U.S. Patent No. 5,256,390, the disclosure of which is hereby incorporated by reference, are also suitable.

Zeolites can be obtained in master batches of pellets of low density polyethylene, polypropylene, ultra high molecular weight polyethylene or polystyrene, containing suitable amounts of zeolite particles, usually 20 wt. % of zeolite particles. When provided in this form, the pellets of resin containing the zeolite particles can be easily mixed with resins used to make the implants or used to make coatings to be applied to the implants, as set forth in U.S. Patent No. 6,582,715, the disclosure of which is hereby incorporated by reference. Typical amounts of zeolite particles incorporated in an implant resin range from 0.01 to 10 wt. %, more preferably 0.01 to 8.0 wt. %, most preferably 0.1 to 5.0 wt. %. The method used to coat an implant is not particularly limited, and can include spraying, painting or dipping. When compounded into PEEK, for example, the PEEK should be protected from sources of moisture and contamination. The compounding can be carried out by blending.

The following clauses refer to particularly preferred embodiments of the present invention.
1. A bone graft substitute comprising an osteogenic agent and particles comprising ion-exchangeable metal cations present in an amount effective for promoting osteogenesis in a patient in need thereof.
2. The bone graft substitute of clause 1 wherein said metal cations are selected from the group consisting of zinc ions, silver ions, copper ions and combinations thereof.
3. The bone graft substitute of clause 1 wherein said metal cation is zinc.
4. The bone graft substitute of clause 1, wherein said particles comprise a zeolite.
5. The bone graft substitute of clause 1, wherein said osteogenesis agent is selected from the group consisting of osteoinductive agents, osteoconductive agents, and combinations thereof.
6. The bone graft substitute of clause 5, wherein said osteoinductive agent is selected from the group consisting of bone morphogenetic protein, demineralized bone matrix, osteoinductive growth factor, osteoblast, and stem cell.
7. The bone graft substitute of clause 5, wherein said osteoconductive agent is selected from the group consisting of collagen-based scaffolds, glass-based scaffolds, silicate-based scaffolds, ceramic-based substitutes, polymer-based substitutes, allografts, calcium phosphates, tricalcium phosphate, fluorapatite, calcium sulfate, demineralized bone matrix and combinations thereof.
8. An implant comprising an osteogenerative agent and particles comprising ion-exchangeable metal cations present in an amount effective for promoting osteogenesis in a patient in need thereof.
9. The implant of clause 8, wherein said metal cations are selected from the group consisting of zinc ions, silver ions, copper ions and combinations thereof.
10. The implant of clause 8, wherein said metal cation is zinc.
11. The implant of clause 8, wherein said particles comprise a zeolite.
12. The implant of clause 8, wherein said osteogenesis agent is selected from the group consisting of osteoinductive agents, osteoconductive agents, and combinations thereof.
13. The implant of clause 8, wherein said implant comprises synthetic bone.
14. The implant of clause 8, where said implant is an interbody spinal cage.
15. A method for modulating bone formation and mineralization, comprising implanting in a subject a bioactive implant comprising an effective amount of a bone morphogenetic protein modulating agent and ion-exchangeable metal cations present in an amount effective for promoting osteogenesis in said subject.
16. The method of clause 15, wherein said metal cations are selected from the group consisting of zinc ions, silver ions, copper ions and combinations thereof.
17. The method of clause 15, wherein said metal cation is zinc.
18. The method of clause 15, wherein said particles comprise a zeolite.
19. The method of clause 15, wherein said osteogenesis agent is selected from the group consisting of osteoinductive agents, osteoconductive agents, and combinations thereof.
20. A method for promoting spinal fusion, the method comprising the steps of: identifying a fusion site between adjacent vertebrae; implanting a bone graft at said site; and presenting a source of metal cations at said fusion site in an amount effective for promoting osteogenesis.
21. The method of clause 20, wherein said metal cations are presented in the form of a zeolite comprising ion-exchangeable cations selected from the group consisting of zinc, silver, copper and combinations thereof.
22. A method of selectively up-regulating the expression of bone morphogenetic protein(s) in the tissue of an animal including human, comprising implanting in said animal a bioactive implant comprising an osteogenerative agent and ion-exchangeable metal cations present in an amount effective for upregulating the expression of bone morphogenetic protein(s).
23. An intracorporeal device comprising an osteogenerative agent and particles comprising ion-exchangeable metal cations present in an amount effective for promoting osteogenesis in a patient in need thereof.
24. The intracorporeal device of clause 23, wherein said metal cations are selected from the group consisting of zinc ions, silver ions, copper ions and combinations thereof.
25. The intracorporeal device of clause 23, wherein said metal cation is zinc.
26. The intracorporeal device of clause 23, wherein said particles comprise a zeolite.
27. The intracorporeal device of clause 23, wherein said osteogenesis agent is selected from the group consisting of osteoinductive agents, osteoconductive agents, and combinations thereof.
28. The intracorporeal device of clause 23, wherein said device is a device selected from the group consisting of a dental implant, appliance, peg, post, cap, crown, bridge and bridge reinforcement.

## Claims

1. A bone graft substitute comprising an osteogenic agent and particles comprising ion-exchangeable metal cations present in an amount effective for promoting osteogenesis in a patient in need thereof.

2. An implant comprising an osteogenerative agent and particles comprising ion-exchangeable metal cations present in an amount effective for promoting osteogenesis in a patient in need thereof.

3. The bone graft substitute of claim 1 or the implant of claim 2, wherein said metal cations are selected from the group consisting of zinc ions, silver ions, copper ions and combinations thereof.

4. The bone graft substitute of claim 1 or the implant of claim 2, wherein said particles comprise a zeolite.

5. The bone graft substitute of claim 1 or the implant of claim 2, wherein said osteogenesis agent is selected from the group consisting of osteoinductive agents, osteoconductive agents, and combinations thereof.

6. A method for modulating bone formation and mineralization, comprising implanting in a subject a bioactive implant comprising an effective amount of a bone morphogenetic protein modulating agent and ion-exchangeable metal cations present in an amount effective for promoting osteogenesis in said subject.

7. The method of clause 6, wherein said metal cations are selected from the group consisting of zinc ions, silver ions, copper ions and combinations thereof.

8. The method of clause 6, wherein said particles comprise a zeolite.

9. The method of clause 6, wherein said osteogenesis agent is selected from the group consisting of osteoinductive agents, osteoconductive agents, and combinations thereof.

10. A method for promoting spinal fusion, the method comprising the steps of: identifying a fusion site between adjacent vertebrae; implanting a bone graft at said site; and presenting a source of metal cations at said fusion site in an amount effective for promoting osteogenesis.

11. The method of clause 10, wherein said metal cations are presented in the form of a zeolite comprising ion-exchangeable cations selected from the group consisting of zinc, silver, copper and combinations thereof.

12. A method of selectively up-regulating the expression of bone morphogenetic protein(s) in the tissue of an animal including human, comprising implanting in said animal a bioactive implant comprising an osteogenerative agent and ion-exchangeable metal cations present in an amount effective for upregulating the expression of bone morphogenetic protein(s).

13. An intracorporeal device comprising an osteogenerative agent and particles comprising ion-exchangeable metal cations present in an amount effective for promoting osteogenesis in a patient in need thereof.

14. The intracorporeal device of clause 13, wherein said metal cations are selected from the group consisting of zinc ions, silver ions, copper ions and combinations thereof.

15. The intracorporeal device of clause 13, wherein said device is a device selected from the group consisting of a dental implant, appliance, peg, post, cap, crown, bridge and bridge reinforcement.
